# EUROPÄISCHE PATENTANMELDUNG

(11) **EP 4 647 025 A1**
(43) Veröffentlichungstag der Anmeldung: **12.11.2025**
(21) Anmeldenummer: 25170071.2
(22) Anmeldetag: 11.04.2025
(51) Int. Cl.: A61B 18/14, A61B 17/00, A61B 18/00

(54) **ABLATIONSKATHETERANORDNUNG UND VORRICHTUNG ZUR ABLATION VON GEWEBE**

(30) Priorität: 08.05.2024 DE 102024112952
(71) Anmelder: Stockert GmbH, 79111 Freiburg im Breisgau (DE)
(72) Erfinder: KEES, Fabian, 79110 Freiburg im Breisgau (DE); KOCH, Fritz, 79100 Freiburg (DE)
(74) Vertreter: Frenkel, Matthias Alexander

(57) **Zusammenfassung**

Die vorliegende Erfindung betrifft eine Ablationskatheteranordnung und eine Vorrichtung zur Ablation von Gewebe. Ein Ausführungsbeispiel einer Ablationskatheteranordnung weist mindestens ein inneres Katheter-Modul mit einem ersten Schaft und einer an dem ersten Schaft befestigten ersten flexiblen Außenstruktur, die ausgebildet ist, einen kollabierten Zustand und expandierten Zustand anzunehmen. Die Ablationskatheteranordnung weist ein äußeres Katheter-Modul mit einem zweiten Schaft und einer an dem zweiten Schaft befestigten zweiten flexiblen Außenstruktur, die ausgebildet ist, einen Ausgangszustand und einen Endzustand anzunehmen. Der zweite Schaft ist ausgebildet, ein inneres Katheter-Modul des mindestens einen inneren Katheter-Moduls in dem zweiten Schaft derart aufzunehmen, dass die zweite flexible Außenstruktur den Ausgangszustand annimmt, wenn sich die erste flexible Außenstruktur in dem kollabierten Zustand befindet, und dass die zweite flexible Außenstruktur den Endzustand annimmt, wenn die erste flexible Außenstruktur sich in dem expandierten Zustand befindet.

## Beschreibung

Die vorliegende Erfindung betrifft eine Ablationskatheteranordnung und eine Vorrichtung zur Ablation von Gewebe.

In den vergangenen Jahren hat sich die Behandlung von Gewebe mittels gepulster elektrischer Felder als eine zunehmend relevante klinische Technik etabliert. Die Verwendung von kurzen Hochspannungsimpulsen und die damit verbundenen hohen elektrischen Feldstärken, die auf das Gewebe einwirken, sind jedoch bereits seit mehr als vier Jahrzehnten Gegenstand intensiver Forschung. Diese Anwendungsmethode wird als nicht-thermisches Verfahren kategorisiert, da sie auf der Abgabe kurzer Pulse mit hoher Spannungsamplitude basiert, die ein lokal starkes elektrisches Feld im Bereich von mehreren Hundert Volt pro Zentimeter zwischen aktiven Elektrodenpaaren erzeugen. Diese Feldstärke führt zur Bildung von Poren in den Zellmembranen. Überschreitet das elektrische Feld einen bestimmten Schwellenwert, der für die Bildung von Poren in den Lipiddoppelschichten der Zellmembranen erforderlich ist, und ist das Gewebe diesem Feld über einen kritischen Zeitraum ausgesetzt, wird die Elektroporation irreversibel. Die Poren bleiben permanent geöffnet, was letztendlich zum programmierten Zelltod (Apoptose) der betroffenen Zelle führt.

Unter anderem zur Behandlung von Herzrhythmusstörungen hat sich die gepulste Feldablation als vielversprechendes Behandlungsverfahren herausgestellt. Herzrhythmusstörungen, wie z. B. Vorhofflimmern, treten auf, wenn erkrankte Bereiche des Herzgewebes abnormale elektrische Signale bilden oder weiterleiten. Dadurch wird der normale Herzschlag gestört und ein asynchroner Rhythmus verursacht. Eine mögliche Behandlungsform dieser Arrhythmien ist die interventionelle Unterbindung der elektrischen Signalweiterleitung der Störsignale. Dabei kann durch selektive Ablation des Herzgewebes mittels Energiezufuhr über einen Katheter eine nicht-leitende Läsion geformt werden, die die Ausbreitung unerwünschter elektrischer Signale unterbindet. Bei einer speziellen Art von Arrhythmie, dem Vorhofflimmern, liegt der Ursprung der elektrischen Störsignale in den Lungenvenen (Pulmonalvenen), die in das linke Atrium des Herzens münden. Mittels Katheterablation (Pulmonalvenenisolation) werden zur Behandlung hierbei gezielt die Pulmonalvenen elektrisch vom linken Atrium isoliert.

Die renale Denervation ist eine Methode, bei der verstärkte Sympathikusaktivitäten unterdrückt werden, um Bluthochdruck oder andere kardiovaskuläre Störungen und chronische Nierenerkrankungen zu behandeln. Dies geschieht über einen minimalinvasiven Eingriff, bei dem mit Hilfe eines speziellen Katheters die Nervenleitungen rund um die Nierenarterien unterbunden werden. Der Katheter wird dabei in die Nierenarterie eingeführt, und die außerhalb verlaufenden Nerven werden in der Regel durch die Gefäßwand hindurch mittels thermischer oder chemischer Ablation verödet.

Mehrere klinische Studien haben bereits gezeigt, dass eine kombinierte Behandlung aus Pulmonalvenenisolation und renaler Denervation zu einer Reduzierung des Wiederauftretens von Vorhofflimmern nach Ablation führen kann.

Zur Durchführung der kombinierten Behandlung aus Pulmonalvenenisolation und renaler Denervation ist es nötig, eine Behandlung in verschiedenen Bereichen des Körpers eines Patienten, insbesondere in verschiedenen Organen und/oder Gefäßen, durchzuführen. Hierfür werden herkömmlich separate Vorrichtungen verwendet. Die gleichen oder ähnliche Probleme bestehen bei der Kombination anderer Behandlungsverfahren. Auch hier müssen oftmals unterschiedliche Vorrichtungen zur Kombination der Behandlungsverfahren eingesetzt werden.

In dem Dokument US 10,016,233 B2 wird ein System für die kombinierte Behandlung von Vorhofflimmern durch kardiale Ablation sowie renale Denervation beschrieben. Die Ablation erfolgt hierbei mit Hilfe herkömmlicher Ablationsverfahren. Zudem sind zwei unabhängige Katheter zur Durchführung der kardialen Ablation und der renalen Denervation notwendig.

Das Dokument US 10,517,672 B2 beschreibt ein System, das einen HochspannungsGenerator sowie einen Ballon-Katheter-System aufweist und darüber IRE-Pulse abgeben kann. Dies geschieht an der inneren Wand der Nierenarterien, um durch sie hindurch die außen liegenden Nervenstränge zu abladieren und zu veröden.

Es besteht jedoch weiter ein Bedarf an einer Anordnung und einem System, die flexibel bei der Kombination mehrerer Behandlungsverfahren einsetzbar sind. Insbesondere besteht ein Bedarf an flexibel in verschiedenen Behandlungsverfahren einsetzbaren Kathetern.

Gemäß einem ersten Aspekt wird eine Ablationskatheteranordnung vorgeschlagen. Die Ablationskatheteranordnung weist mindestens ein inneres Katheter-Modul auf. Die Ablationskatheteranordnung weist ein äußeres Katheter-Modul auf. Das mindestens eine innere Katheter-Modul weist (jeweils) mindestens einen ersten Schaft und eine an dem ersten Schaft befestigte erste flexible Außenstruktur auf. Die erste flexible Außenstruktur ist ausgebildet, einen kollabierten Zustand und expandierten Zustand anzunehmen. Das äußere Katheter-Modul weist einen zweiten Schaft und eine an dem zweiten Schaft befestigte zweite flexible Außenstruktur auf. Die zweite flexible Außenstruktur ist ausgebildet, einen Ausgangszustand und einen Endzustand anzunehmen. Der zweite Schaft (des äußeren Katheter-Moduls) ist ausgebildet, ein inneres Katheter-Modul des mindestens einen inneren Katheter-Moduls in dem zweiten Schaft aufzunehmen. Der zweite Schaft ist ausgebildet, ein inneres Katheter-Modul des mindestens einen inneren Katheter-Moduls in dem zweiten Schaft derart aufzunehmen, dass die zweite flexible Außenstruktur den Ausgangszustand annimmt, wenn sich die erste flexible Außenstruktur in dem kollabierten Zustand befindet, und dass die zweite flexible Außenstruktur den Endzustand annimmt, wenn die erste flexible Außenstruktur sich in dem expandierten Zustand befindet.

Der Ausgangszustand kann auch als Ausgangsformzustand und der Endzustand kann auch als Endformzustand bezeichnet werden, da beide Zustände eine Form des äußeren Katheter-Moduls, insbesondere der zweiten flexiblen Außenstruktur des äußeren Katheter-Moduls, betreffen oder bezeichnen können. Das mindestens eine innere Katheter-Modul und das äußere Katheter-Modul können von einem unterschiedlichen Typ sein oder einen unterschiedlichen Katheter-Typ betreffen. Das mindestens eine innere Katheter-Modul und das äußere Katheter-Modul können beispielsweise eine unterschiedliche Außenstruktur aufweisen. Eines oder mehrere, insbesondere jedes, des mindestens einen inneren Katheter-Moduls kann von einem gleichen Typ sein oder einen gleichen Katheter-Typ betreffen. Dennoch können sich eines oder mehrere, insbesondere jedes, des mindestens einen inneren Katheter-Moduls voneinander, beispielsweise in seiner Dimensionierung und/oder in seiner Größe und/oder in seinem expandierten Zustand, unterscheiden. Zwischen dem Ausgangszustand und dem Endzustand können ein oder mehrere Zwischenzustände existieren. Die Zwischenzustände der zweiten flexiblen Außenstruktur können entsprechend einem Expansionsgrad der ersten flexiblen Außenstruktur angenommen werden.

Das mindestens eine innere Katheter-Modul kann auf mindestens einem inneren Katheter basieren oder mindestens einem inneren Katheter entsprechen. Das äußere Katheter-Modul kann auf mindestens einem äußeren Katheter basieren oder mindestens einem äußeren Katheter entsprechen. Zumindest das äußere Katheter-Modul kann auf einem Ablationskatheter basieren oder einem Ablationskatheter entsprechen-

Das mindestens eine innere Katheter-Modul kann als ein Ballon-Katheter-Modul ausgebildet sein oder ein Ballon-Katheter-Modul aufweisen. Das Ballon-Katheter-Modul kann auf einem Ballon-Katheter basieren oder einem Ballon-Katheter entsprechen. Die erste flexible Außenstruktur kann als eine flexible Membran ausgebildet sein oder eine flexible Membran aufweisen. In diesem Fall kann die flexible Membran in dem kollabierten Zustand eine beispielsweise schlauchförmige Form annehmen. In dem expandierten Zustand kann die flexible Membran eine beispielsweise ballonförmige Form annehmen. In dem einen oder den mehreren Zwischenzuständen kann die flexible Membran ein ballonartige oder ballonförmige Form annehmen. Beispielsweise können, ausgehend von dem Ausgangszustand, die Zwischenzustände bis zu dem Endzustand eine zunehmend ballonförmige Form annehmen.

Das innere Katheter-Modul kann ein proximales Ende und ein distales Ende aufweisen. Die erste flexible Außenstruktur, insbesondere die flexible Membran, kann zwischen dem distalen Ende und dem proximalen Ende angeordneten sein. Das äußere Katheter-Modul kann ein proximales Ende und ein distales Ende aufweisen. Die zweite flexible Außenstruktur kann zwischen dem distalen Ende und dem proximalen Ende angeordnet sein.

Auf einer Außenseite der zweiten flexiblen Außenstruktur können mehrere Elektroden angeordnet sein. Die zweite flexible Außenstruktur kann mehrere Rippen aufweisen oder durch mehrere Rippen gebildet sein. An jeder der mehreren Rippen kann mindestens eine Elektrode angeordnet oder ausgebildet sein.

Die zweite flexible Außenstruktur kann beispielsweise korbförmig ausgebildet sein. Beispielsweise können die mehreren Rippen korbförmig verlaufen oder eine Korbform bilden. Beispielsweise können die mehreren Rippen von einem proximalen Bereich des äußeren Katheter-Moduls, insbesondere des zweiten Schafts, zu einem distalen Bereich des äußeren Katheter-Moduls, insbesondere des zweiten Schafts verlaufen.

In dem zweiten Schaft kann ein Innenschaft derart angeordnet und ausgebildet sein, jeweils ein inneres Katheter-Modul des mindestens einen inneren Katheter-Moduls aufzunehmen. Die Dimensionen des Innenschafts, insbesondere ein Querschnitt eines Innenraums des Innenschafts und/oder eine Länge des Innenraums, können so bemessen sein, dass eines des mindestens einen inneren Katheter-Moduls in dem Innenschaft aufnehmbar ist. Beispielsweise kann als das mindestens eine innere Katheter-Modul eine Vielzahl von inneren Katheter-Modulen vorgesehen oder bereitgestellt sein. In diesem Fall kann jeweils ein inneres Katheter-Modul der Vielzahl von inneren Katheter-Modulen in den Innenschaft des äußeren Katheter-Moduls einführbar sein. Wird das eingeführte innere Katheter-Modul, insbesondere dessen erste flexible Außenstruktur, von seinem kollabierten in seinen expandierten Zustand gebracht, nimmt das äußere Katheter-Modul, insbesondere dessen zweite flexible Außenstruktur, einen Endzustand an, der von dem expandierten Zustand abhängt oder der dem expandierten Zustand zumindest nahezu entspricht. Wird nun ein anderes inneres Katheter-Modul der Vielzahl von inneren Katheter-Module in den Innenschaft eingebracht und nimmt seinen expandierten Zustand an, der von dem vorherigen expandierten Zustand abweicht, so nimmt das äußere Katheter-Modul einen Endzustand an, der von dem expandierten Zustand abhängt oder der dem expandierten Zustand zumindest nahezu entspricht und der somit von dem vorherigen Endzustand abweicht. Auf diese Weise können mit der gleichen Ablationskatheteranordnung verschiedene Endzustände, insbesondere Formzustände oder Formen, angenommen werden. Somit können durch Einbringen unterschiedlicher innerer Katheter-Module unterschiedliche Endzustände für das äußere Katheter-Modul, insbesondere dessen zweiter flexibler Außenstruktur, bewirkt werden. Ebenso können durch Einbringen unterschiedlicher innerer Katheter-Module unterschiedliche Zwischenzustände für das äußere Katheter-Modul, insbesondere dessen zweiter flexibler Außenstruktur, bewirkt werden.

An einem distalen Ende des äußeren Katheter-Moduls kann eine distale Elektrode angeordnet sein. An einem proximalen Ende des äußeren Katheter-Moduls kann eine proximale Elektrode angeordnet sein. Die distale Elektrode und/oder die proximale Elektrode kann/können ringförmig ausgebildet sein. Somit kann das äußere Katheter-Modul proximal und distal von der zweiten flexiblen Außenstruktur jeweils eine an dem zweiten Schaft angebrachte oder angeordnete ringförmige Elektrode aufweisen.

Gemäß einem zweiten Aspekt wird eine Vorrichtung zur Ablation eines Gewebes eines Patienten vorgeschlagen. Die Vorrichtung weist eine Ablationskatheteranordnung gemäß dem ersten Aspekt auf. Die Vorrichtung weist eine mit der Ablationskatheteranordnung verbundene oder verbindbare Signalgeneratoranordnung auf. Die Vorrichtung weist eine mit der Ablationskatheteranordnung und/oder der Signalgeneratoranordung verbundene oder verbindbare Steuer- und Auswerteeinheit auf.

Die Steuer- und Auswerteeinheit kann dazu eingerichtet sein, verschiedene Messungen und/oder Behandlungsvorgänge durchzuführen und/oder zu instruieren und/oder zu steuern. Beispielsweise kann die Steuer- und Auswerteeinheit ausgebildet sein, eine Gewebeimpedanzbestimmung und/oder eine Ablation und/oder eine Denervationsmessung und/oder eine Denervation durchzuführen und/oder zu instruieren und/oder zu steuern. Die Steuer- und Auswerteeinheit kann beispielsweise ausgebildet sein, eine Gewebeimpedanzbestimmung und, beispielsweise anschließend, eine Ablation durchzuführen. Zusätzlich oder alternativ kann die Steuer- und Auswerteeinheit ausgebildet sein, eine Denervationsmessung und, beispielsweise anschließend, eine Denervation durchzuführen.

Die Signalgeneratoranordnung kann einen ersten Signalgenerator zur Erzeugung eines Radiofrequenz (RF) -Signals und einen zweiten Signalgenerator zur Erzeugung eines Signals für eine gepulste Feldablation (PFA) aufweisen. Das RF-Signal kann für eine herkömmliche thermische Ablation genutzt werden. Das Signal für die PFA (=PFA-Signal) kann für eine irreversible Elektroporation genutzt werden.

Die Steuer- und Auswerteeinheit kann eingerichtet sein, über eine Elektrodenkonstellation insbesondere der Ablationskatheteranordnung ein erstes elektrisches Signal in das Gewebe abzugeben. Die Steuer- und Auswerteeinheit kann eingerichtet sein, ein erstes elektrisches Signal in das Gewebe abzugeben und ein zweites elektrisches Signal über die Elektrodenkonstellation der insbesondere Ablationskatheteranordnung aus dem Gewebe zu empfangen. Unter der Elektrodenkonstellation kann eine paarweise Zuordnung von Elektroden verstanden werden. Die Signalgenerator-Auswerteeinheit kann eingerichtet sein, Elektrodenpaare und/oder Elektrodenkonstellationen anzusteuern und/oder zwischen Elektroden-(paaren) und/oder Elektrodenkonstellationen zu schalten.

Eine Elektrodenkonstellation kann flexibel definiert/gebildet/zugeordnet werden aus der proximalen und/oder der distalen Elektrode und/oder einer oder mehreren der Vielzahl von auf der zweiten flexiblen Außenstruktur angeordneten Elektroden der Ablationskatheteranordnung. Je nach Einsatzbereich können beliebige Elektrodenkonstellationen gebildet werden.

Je nach Einsatzbereich kann das erste elektrische Signal als elektrisches Stromsignal und das zweite elektrische Signal als elektrisches Spannungssignal ausgebildet sein oder umgekehrt. Beispielsweise kann die Steuer- und Auswerteeinheit dazu eingerichtet sein, aus dem in das Gewebe geleiteten elektrischen Stromsignal (=dem ersten elektrischen Signal) und aus dem Gewebe empfangenen elektrischen Spannungssignal (=zweiten elektrischen Signal) zumindest eine, insbesondere lokale, Gewebeimpedanz zu bestimmen. Die Steuer- und Auswerteeinheit kann dazu eingerichtet sein, mittels, insbesondere (zeitlich) nacheinander gewählten/geschalteten, zumindest zwei (anzusteuernden) Elektrodenkonstellationen/Elektrodenpaaren/Elektroden, zumindest zwei, insbesondere lokale, Gewebeimpedanzen zu bestimmen.

Die Steuer- und Auswerteeinheit kann ausgebildet sein, den ersten Signalgenerator und/oder den zweiten Signalgenerator zur Abgabe eines Signals zu steuern / anzusteuern. Auf diese Weise kann nach Bedarf das RF-Signal und/oder PFA-Signal ausgegeben werden.

Die Steuer- und Auswerteeinheit kann ausgebildet sein, in Abhängigkeit von mindestens einer Elektroden-Temperatur den ersten Signalgenerator oder den zweiten Signalgenerator zur Abgabe eines Signals, beispielsweise eines RF-Signals, anzusteuern. Beispielsweise kann die Steuer- und Auswerteeinheit ausgebildet sein, falls die Elektroden-Temperatur einen Temperatur-Grenzwert unterschreitet, den ersten Signalgenerator anzusteuern und, falls die Elektroden-Temperatur den Temperatur-Grenzwert annimmt oder überschreitet, den zweiten Signalgenerator zur Abgabe eines Signals, beispielsweise eines PFA-Signals, anzusteuern.

Weitere Merkmale, Eigenschaften, Vorteile und mögliche Abwandlungen werden für einen Fachmann anhand der nachstehenden Beschreibungen deutlich, in der auf die beigefügten Zeichnungen Bezug genommen ist.
Figur 1 ist eine schematische Darstellung eines bipolaren IRE-Impulses entsprechend einer beispielhaften Ausführung.
Figur 2 ist eine schematische Darstellung eines Puls Protokolls mit mehreren Serien bzw. Bursts bipolarer Impulse entsprechend einer beispielhaften Ausführung.
Figur 3 zeigt eine schematische Darstellung eines Puls Protokolls für eine kombinierte Behandlung mit RF und gepulster Feldablation mit mindestens einer Serie von RF-Signalen und mindestens einer Serie bzw. Burst bipolarer IRE-Pulse entsprechend einer beispielhaften Ausführung.
Figur 4 zeigt eine schematische Darstellung eines äußeren Katheter-Moduls einer Ablationskatheteranordnung in kollabierter Form.
Figur 5 zeigt eine schematische Darstellung eines inneren Katheter-Moduls in expandierter Form.
Figur 6 zeigt eine schematische Darstellung einer Ablationskatheteranordnung gemäß einem Ausführungsbeispiel mit dem äußeren Katheter-Modul aus Figur 4 und dem inneren Katheter-Modul aus Figur 5.
Figur 7 zeigt eine schematische Darstellung eines inneren Katheter-Moduls in expandierter Form.
Figur 8 zeigt eine schematische Darstellung einer Ablationskatheteranordnung gemäß einem Ausführungsbeispiel mit dem äußeren Katheter-Modul aus Figur 4 und dem inneren Katheter-Modul aus Figur 7.
Figur 9 zeigt eine schematische Darstellung eines äußeren Katheter-Moduls gemäß einer Variante des Katheter-Moduls aus Figur 4 in kollabierter Form.
Figur 10 zeigt eine schematische Darstellung einer Ablationskatheteranordnung gemäß einem Ausführungsbeispiel mit dem äußeren Katheter-Modul aus Figur 9 und dem inneren Katheter-Modul aus Figur 7.
Figur 11 zeigt schematische Verschaltungen von Elektroden der Ablationskatheteranordnung aus Figur 6 zur lokalen Impedanzmessung.
Figur 12 zeigt schematische Verschaltungen von Elektroden der Ablationskatheteranordnung aus Figur 6 zur Stimulation und Messungen der Nervenaktivität.
Figur 13 zeigt ein Flussdiagramm von Verfahrensschritten zur Durchführung einer Denervation.

Die irreversible Elektroporation (IRE) ist primär ein nicht-thermisches Verfahren, welches nur eine geringe elektrische Energie nutzt und damit eine Erhöhung der Gewebetemperatur um nur einige °C bewirkt. Dies grenzt es deutlich von der herkömmlichen RF-Ablation (RF: Radiofrequenz) ab, bei der die Gewebetemperatur um 20 bis 70 °C steigt und Zellen durch Hitze zerstört werden. Bei der IRE werden in der Regel bipolare Pulse eingesetzt, d.h. eine Kombination aus positiven und negativen elektrischen Pulsen, um Muskelkontraktionen, die üblicherweise bei Anwendung von Gleichspannung entstehen, weitestgehend zu vermeiden. Diese Pulse können zwischen zwei bipolaren Elektroden eines Katheters oder zwischen einer Katheterelektrode und einer Körperoberflächenelektrode, die üblicherweise auf dem Rücken des Patienten angebracht wird, angelegt werden.

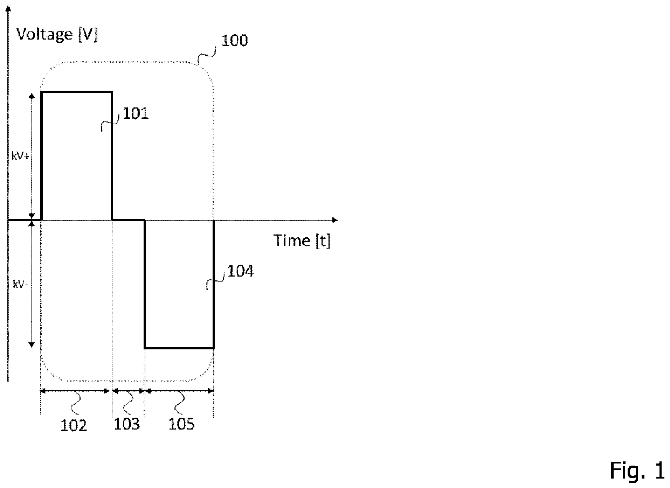

Figur 1 ist eine schematische Darstellung eines biphasischen IRE-Pulses entsprechend einer beispielhaften Ausführung. Sie zeigt die Spannung V des biphasisch PFA-Pulses 100 als Funktion der Zeit t in einem IRE-Ablationsverfahren. Die vorliegenden beispielhaften Ausführungsformen beziehen sich auf einen IRE-Generator, der als Spannungsquelle konfiguriert ist. Folglich werden die IRE-Signale hier in Form ihrer Spannungen beschrieben. Der biphasische IRE-Puls umfasst einen positiven Puls 101 und einen negativen Puls 104, wobei sich die Begriffe "positiv" und "negativ" auf eine unabhängig gewählte Polarität zweier für die Ablation angesteuerten Elektroden beziehen, zwischen denen der biphasische Puls angelegt wird. Die Amplitude des positiven Pulses 101 wird mit kV+ bezeichnet und dauert über die Zeit 102 an. Analog wird die Amplitude des negativen Pulses 104 mit kV- bezeichnet und weist eine zeitliche Breite 105 auf. Zwischen den beiden Puls-Phasen 101 und 104 kann eine Verzögerungszeit 103 liegen. Sowohl die beiden zeitlichen Puls-Breiten 102 und 105 als auch die Amplitude kV+ und kV- sind unabhängig voneinander konfigurierbar und können daher in einer exemplarischen Ausführung variieren.

Der in Figur 1 schematisch gezeigte bipolare Puls 100 kann von der Signalgeneratoranordnung generiert werden. Die Formate, welche die Eigenschaften des bipolaren Pulses 100 bestimmen, können durch einen Nutzer vordefiniert worden. Die Werte der Pulsauslenkung kV+, kV- des positiven 101 und negativen Pulses 104 können beispielhaft ±500kV sein. Der dritte zeitliche Abstand 103 zwischen dem positiven 101 und dem negativen Puls 104 kann beispielhaft 2,5µs sein. Die Pulsbreite 102 des positiven Pulses 101 kann sich von der Pulsbreite 105 des negativen Pulses 104 unterscheiden. Der Unterschied der Pulsbreiten ist in der Figur 1 nicht dargestellt.

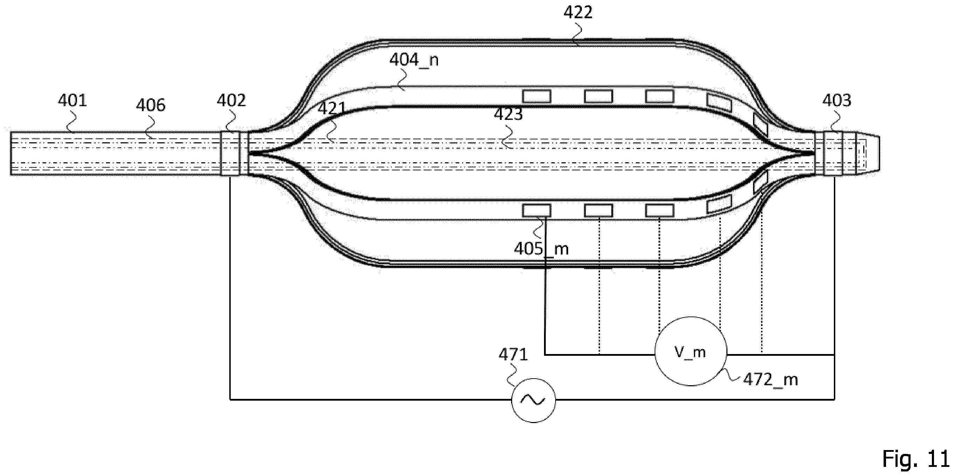

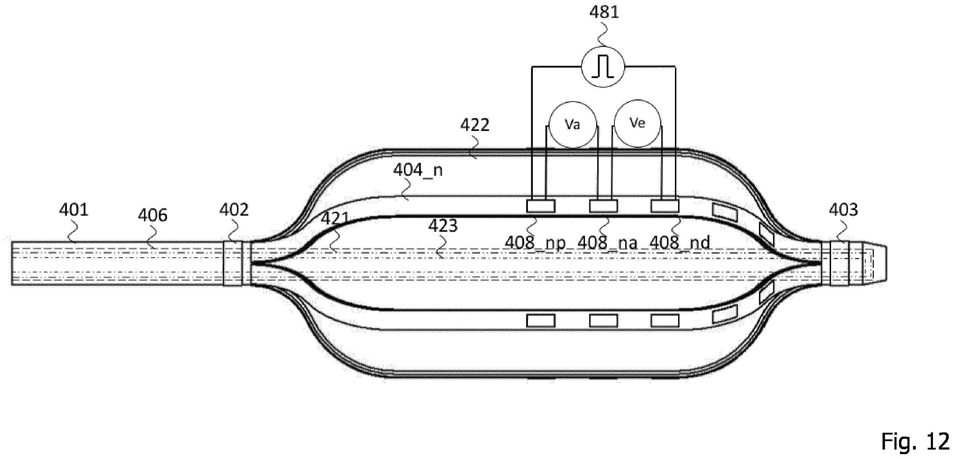

Die erzeugte Puls kann, wie nachfolgend u. a. in Bezug auf die Figuren 11 und 12 erläutert werden wird, verwendet werden.

Damit die IRE-Pulse Poren im Gewebe erzeugen, muss die durch die Pulse definierte elektrische Feldstärke E am Gewebe zwischen einem Paar aus mindestens zwei Elektroden einen gewebeabhängigen Schwellenwert Eth überschreiten. So liegt der Schwellenwert für Herzzellen beispielsweise bei etwa 500 V/cm, während er für Knochen bei 3000 V/cm liegt. Diese Unterschiede in den Schwellenfeldstärken ermöglichen eine selektive Anwendung von IRE in verschiedenen Geweben. Um die erforderliche Feldstärke zu erreichen, hängt die an ein Elektrodenpaar anzulegende Spannung sowohl vom Zielgewebe als auch vom Abstand zwischen den Elektroden und von der Elektrodengröße selbst ab. Gleichermaßen beeinflussen diese Parameter auch den thermischen Energieeintrag während der Ablation und damit die Temperaturspitzen, die an dem zu behandelnden Gewebe auftreten können. Die angelegten Spannungen können mehrere Kilovolt erreichen, was wesentlich höher ist als die typischen Spannungen von 10-200 V bei der thermischen RF-Ablation.

Der bipolare Pulsed Field Ablation-Puls (bipolare PFA-Puls) für IRE enthält einen positiven und einen negativen Puls (wie beispielhaft in Figur 1 gezeigt), die zwischen zwei Elektroden mit einer Pulsbreite von 1 bis 5 µs und einem Abstand zwischen den positiven und negativen Pulsen von 1 bis 5 µs angelegt werden. Die bipolaren Pulse werden zu Pulsfolgen zusammengesetzt, wobei jede Folge über hundert bipolare Pulse mit einem Puls-zu-Puls-Abstand von 1 bis 10 ms umfassen kann. Die Pulsfolgen bilden jeweils einen Burst, wobei sich das gesamte Pulspaket der IRE-Ablation aus 1-20 Bursts/Bursteinheiten zusammensetzt, die jeweils ein Burst-zu-Burst-Abstand von 1 bis 1000 ms aufweisen. Die Gesamtdauer einer Ablation kann bis zu 10 s betragen.

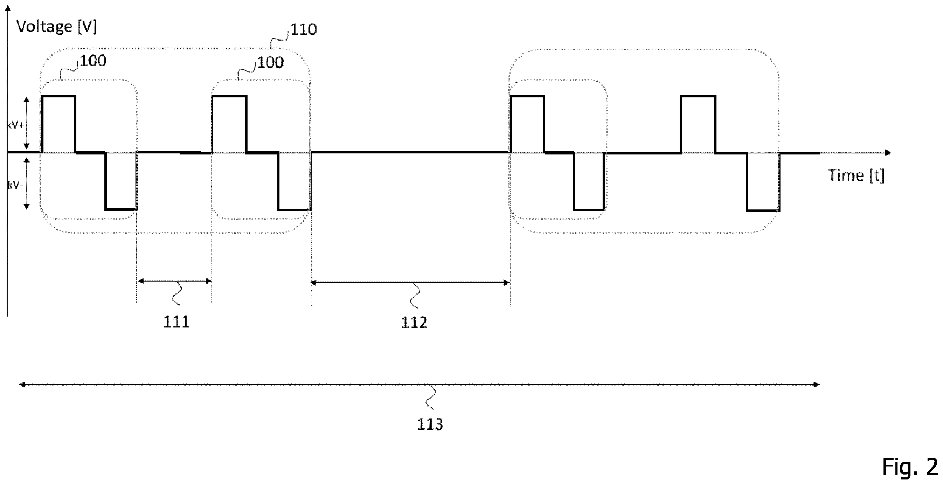

Figur 2 ist eine schematische Darstellung eines Puls-Protokolls mit mehreren Bursts biphasischer Pulse entsprechend einer beispielhaften Ausführung. Über die Dauer der kompletten IRE-Prozedur 113 werden die Pulse 100 in Form eines oder mehrerer Bursts bzw. Pulspakete 110 abgegeben. Jeder Burst 110 umfasst dabei eine definierte Anzahl N an biphasischen Pulsen 100, wobei die Pulse durch ein zeitliches Intervall 111 separiert sind. Zwischen der Abgabe der einzelnen Bursts 110 liegt wiederum eine Verzögerungszeit 112.

Das in Figur 2 gezeigte Puls-Protokoll kann ein elektrisches Signal bilden, das, wie nachfolgend noch genauer beschrieben erzeugt und verwendet werden kann. Das elektrische Signal ist, wie genannt, als Burst-Signalfolge ausgebildet. Zu erkennen sind zwei Bursts, von denen einer mit dem Bezugszeichen 110 versehen ist. Jeder Burst weist mindestens zwei bipolare Pulse 100 auf. Jeder in der Burst-Signalfolge vorkommende bipolare Puls 100 weist beispielhaft die Eigenschaften des Formats aus Figur 1 auf. Die erste Anzahl an Bursts, hier exemplarisch zwei Bursts, die Anzahl der bipolaren Pulse, der zweite zeitliche Abstand 111, und der erste zeitliche Abstand 112 zwischen zwei aufeinanderfolgenden Bursts 110, können von einem Nutzer festgelegt werden. Die zu erkennende Burst-Signalfolge erstreckt sich über eine Dauer 113, die der Dauer der irreversiblen Elektroporation entspricht.

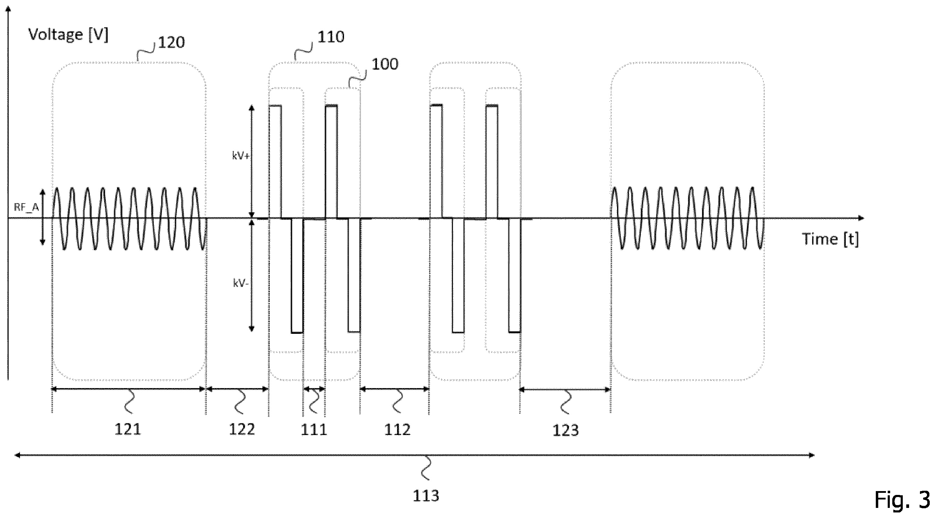

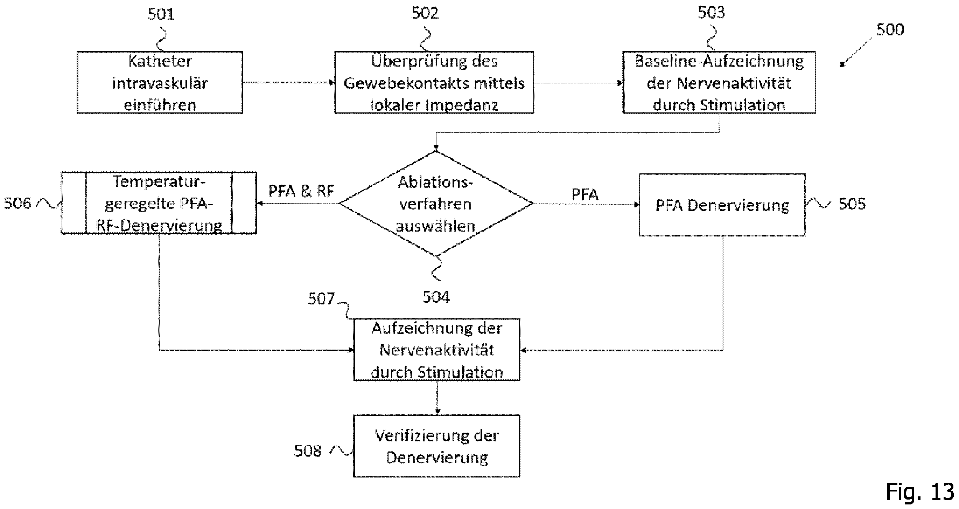

Figur 3 zeigt eine schematische Darstellung eines Prozedur-Protokolls mit mindestens einem Burst biphasischer IRE-Pulse (PFA-Pulse) kombiniert mit mindestens einem RF-Energie Burst 120 entsprechend einer beispielhaften Ausführung, wie sie hierin verwendet werden kann. Eine beispielhafte Verwendung wird in Bezug auf Figur 13 beschrieben werden. Über die Dauer der kompletten kombinierten Prozedur 113 werden die RF-Energie sowie die IRE-Pulse in Form einer oder mehrerer Bursts 120 und 110 abgegeben. Jeder IRE-Burst umfasst dabei eine definierte Anzahl N an bipolaren Pulsen 100, wobei die Pulse durch ein zeitliches Intervall 111 separiert sind. Der RF-Burst ist beschrieben durch ein sinusförmiges Signal mit Amplitude RF_A und einer Dauer 121. Im Anschluss an einen RF-Burst folgt eine Verzögerungszeit 122. Sowohl die Dauer des RF-Bursts als auch die anschließende Verzögerungszeit können basierend auf der aktuell gemessenen Temperatur an den Ablations-Elektroden geregelt werden. Zwischen der Abgabe der einzelnen IRE-Bursts 110 liegt eine Verzögerungszeit 112. Zwischen einem IRE-Burst und einer erneuten Abgabe des RF-Burst liegt eine Verzögerungszeit 123.

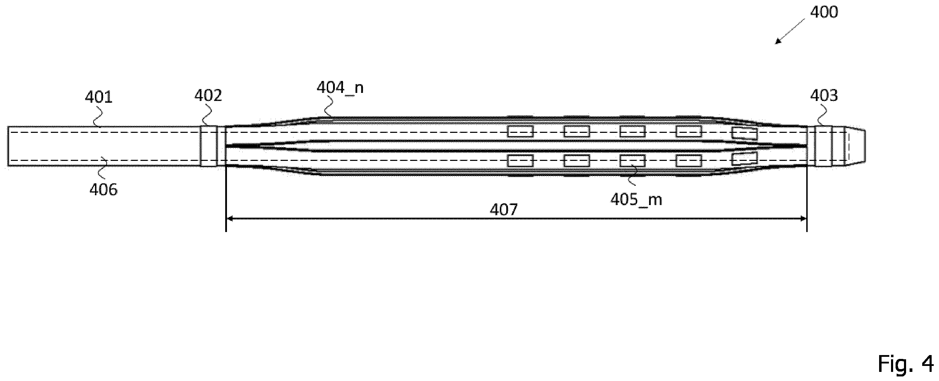

Figur 4 zeigt eine Ausführungsform eines äußeren Katheter-Moduls 400 einer Ablationskatheteranordnung in kollabiertem Zustand, der zur Steuerung in und aus dem Organ/Gefäß des Patienten heraus verwendet wird. Das Katheter-Modul 400 weist einen äußeren, optional steuerbaren, Schaft 401 zur Einführung in ein Organ/Gefäß des Patienten, sowie einen inneren Schaft 406 zur Einführung eines inneren Katheter-Moduls der Ablationskatheteranordnung auf. Der äußere Schaft 401 ist derart ausgeführt, dass er über eine definierte Länge 407 aufgetrennt ist und eine Anzahl n an Rippen/Splines 404_n formt. Auf jeder/jedem dieser Rippen/Splines 404_n ist eine definierte Anzahl m an Elektroden 405_m aufgebracht. Proximal, sowie distal zu den Rippen/Splines 404_n befindet sich jeweils eine ringförmige Elektrode 402 und 403, die fest mit dem äußeren Schaft 401 verbunden sind. Alle Elektroden 405_m, 402 und 403 sind einer äußeren Umgebung ausgesetzt und über eine oder mehrere elektrische Leitungen, die sich vom proximalen Ende über den Schaft 401 hin bis zu den Elektroden erstrecken, elektrisch verbunden sind. Die elektrischen Zuleitungen sind derart abgedeckt, dass sie sowohl untereinander als auch zur äußeren Umgebung elektrisch isoliert sind.

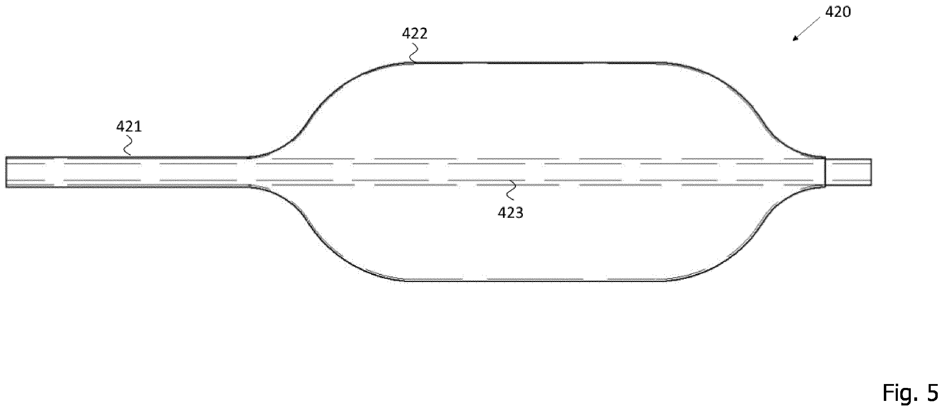

Figur 5 zeigt eine Ausführungsform eines inneren Katheter-Moduls 420 der Ablationskatheteranordnung in einem expandiertem Zustand, der zur Formgebung des äußeren Katheter-Moduls 400 während einer Prozedur verwendet werden kann. Das innere Katheter-Modul 420 weist einen äußeren Schaft 421, einen inneren Schaft 423, sowie eine auf dem äußeren Schaft 423 befestigte expandierte Ballon-Membran 422 auf. Das Expandieren erfolgt über den inneren Schaft 421, der dedizierte Löcher zur Ballon-Membran 422 aufweist.

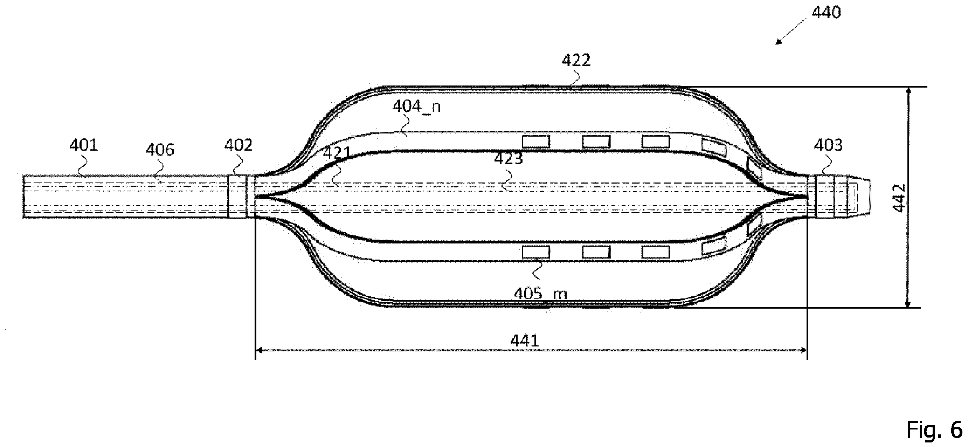

Figur 6 zeigt eine Ausführungsform einer Ablationskatheteranordnung 440, insbesondere eines zusammenhängenden Kathetersystems 440, mit expandiertem Ballon-Element aus Figur 5 als inneres Katheter-Modul und äußerem Katheter-Modul aus Figur 4, wie es während einer Prozedur verwendet werden kann. Das innere Katheter-Modul 420 ist axial in den inneren Schaft 406 des äußeren Katheter-Moduls 400 eingeführt, so dass das Ballon-Element und die Rippen/Splines 404_n bündig zueinander ausgerichtet sind. Durch Expandieren der Ballon-Membran 422 gleicht sich somit die Form eines durch die Rippen/Splines 404_n gebildeten Körbchens des äußeren Katheter-Moduls 400 zusammen mit den darauf angebrachten Elektroden 405_m der definierten Form des Ballon-Elements 422 an. Dies verleiht dem äußeren Katheter-Modul 400 einen effektiven Durchmesser 442 und eine effektive Länge 441. Diese beiden Parameter sind spezifisch über das Ballon-Modul einstellbar und definieren sich über die durchzuführende Prozedur sowie die vorgesehene Geometrie des zu behandelnden Organs/Gefäßes. Die in Figur 6 gezeigte Ausführungsform ist beispielhaft für die Durchführung einer renalen Denervation.

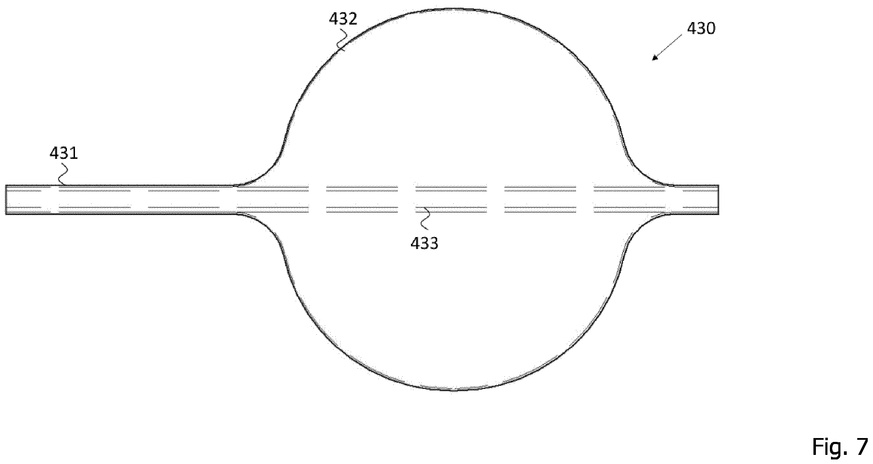

Figur 7 zeigt eine weitere Ausführungsform eines inneren Katheter-Moduls 430 der Ablationskatheteranordnung / des Kathetersystems in expandiertem Zustand, der zur Formgebung eines äußeren Katheter-Moduls während einer Prozedur verwendet werden kann. Das innere Katheter-Modul 430 weist einen äußeren Schaft 431, einen inneren Schaft 433, sowie eine auf dem äußeren Schaft befestigte expandierbare Ballon-Membran 432 auf. Das Expandieren erfolgt über den inneren Schaft, der dedizierte Löcher zur Ballon-Membran 432 aufweist.

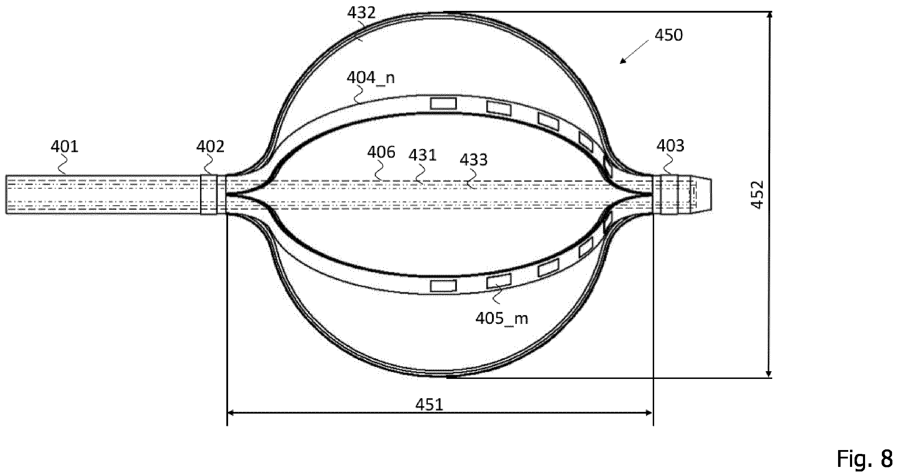

Figur 8 zeigt eine weitere Ausführungsform einer Ablationskatheteranordnung 450, insbesondere eines zusammenhängenden Kathetersystems, mit einem inneren Katheter-Modul 430 mit expandiertem Ballon-Element aus Figur 7 und einem äußeren Katheter-Modul 400 aus Figur 4, wie es während einer Prozedur verwendet werden kann. Das innere Katheter-Modul 430 ist axial in den inneren Schaft 406 des äußeren Katheter-Moduls 400 eingeführt, so dass das Ballon-Element und die Rippen/Splines 404_n bündig zueinander ausgerichtet sind. Durch Expansion der Ballon-Membran 432 gleicht sich somit die Form des durch die Rippen/Splines 404_n gebildeten Körbchens zusammen mit den darauf angebrachten Elektroden 405_m der definierten Form des Ballon-Elements an. Dies verleiht dem äußeren Katheter-Modul 400 einen effektiven Durchmesser 452 und eine effektive Länge 451. Diese beiden Parameter sind spezifisch über das Ballon-Modul einstellbar und definieren sich über die durchzuführende Prozedur sowie die vorgesehene Geometrie des zu behandelnden Organs/Gefäßes. Die in Figur 8 gezeigte Ausführungsform ist beispielhaft für die Durchführung einer Pulmonalvenenisolation.

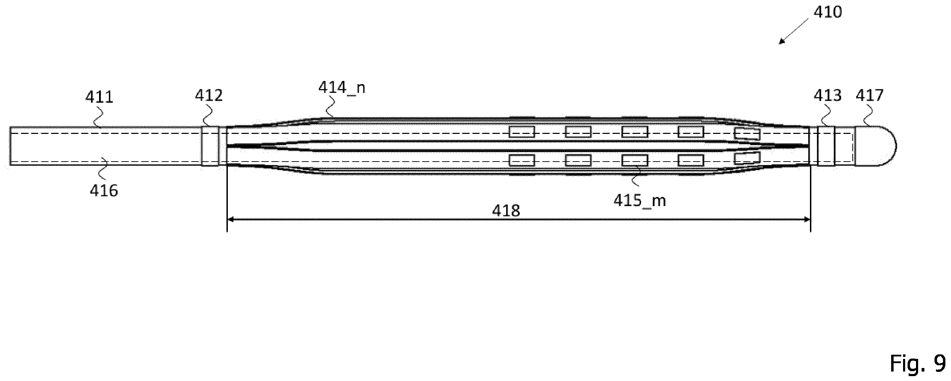

Figur 9 zeigt eine weitere Ausführungsform eines äußeren Katheter-Moduls 410 der Ablationskatheteranordnung in einem kollabiertem Zustand, der zur Steuerung in und aus dem Organ/Gefäß des Patienten heraus verwendet wird. Das äußere Katheter-Modul 410 weist einen äußeren, optional steuerbaren, Schaft 411 zur Einführung in ein Organ/Gefäß des Patienten, sowie einen inneren Schaft 416 zur Einführung des inneren Katheter-Moduls der Anordnung auf. Der äußere Schaft 411 ist derart ausgeführt, dass er über eine definierte Länge 418 aufgetrennt ist und eine Anzahl n an Rippen/Splines 414_n formt. Auf jedem dieser Splines 414_n ist eine definierte Anzahl m an Elektroden 415_m aufgebracht. Proximal, sowie distal zu den Rippen/Splines 414_n befindet sich jeweils eine ringförmige Elektrode 412 und 413, die fest mit dem äußeren Schaft 411 verbunden sind. Zusätzlich ist an der distalen Spitze des Schafts 411 noch eine weitere Elektrode 417 angebracht, die atraumatisch geformt ist. Alle Elektroden sind einer äußeren Umgebung ausgesetzt und über eine oder mehrere elektrische Leitungen, die sich vom proximalen Ende über den Schaft hin bis zu den Elektroden erstrecken, elektrisch verbunden sind. Die Anzahl der elektrischen Zuleitungen sind derart abgedeckt, dass sie sowohl untereinander als auch zur äußeren Umgebung elektrisch isoliert sind.

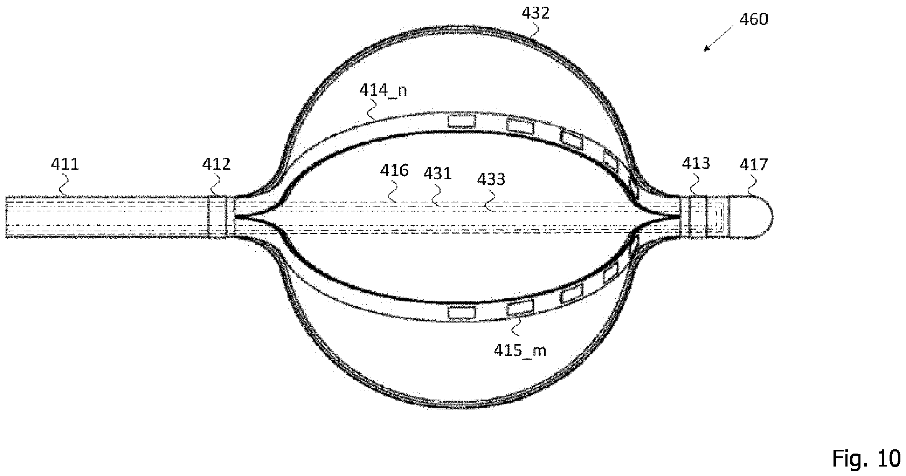

Figur 10 zeigt eine weitere Ausführungsform einer Ablationskatheteranordnung 460, insbesondere eines zusammenhängenden Kathetersystems, mit einem inneren Katheter-Modul 470 mit expandiertem Ballon-Element aus Figur 7 und einem äußeren Katheter-Modul 410 aus Figur 9, wie es während einer Prozedur verwendet werden kann. Die Ausführungsform aus Figur 10 ist eine Variante der Ausführungsform aus Figur 8 und variiert im Vergleich zu der in Figur 8 dargestellten Form lediglich in der Verwendung eines äußeren Katheter-Moduls 410, welches in dieser Ausführungsform dem aus Figur 9 entspricht, d. h. einer Ausführung mit zusätzlicher Tip-Elektrode 417.

Figur 11 zeigt exemplarisch für alle dargestellten Ausführungsformen der Ablationskatheteranordnung einen Mechanismus und eine Ansteuerung zur Bestimmung der lokalen Impedanz des Zielgewebes. Dabei wird ein elektrischer Strom 471 zwischen der proximalen Ring-Elektrode 402 und der distalen Ring-Elektrode 403 angelegt. Um die Impedanz mit Hilfe des Ohmschen Gesetz zu bestimmen, werden die Spannungen 472_m von jeder der Ablations-Elektroden 405_m jedes einzelnen Rippen/Splines 404_n zur distalen Ring-Elektrode 403 gemessen. Dabei entspricht der Gesamtzahl an Spline-Elektroden 405_m über die Anzahl n von Rippen/Splines 404_n hinweg, wodurch sich entsprechend die gleiche Anzahl an Impedanz-Metriken ergeben und die Eigenschaften des Zielgewebes sehr selektiv ermittelt werden können.

Figur 12 zeigt schematisch einen Mechanismus und Ansteuerung zur Bestimmung der Nervenleitfähigkeit. Diese wird einmal vor der Ablation als Ausgangswert gemessen und erneut im Anschluss an die Prozedur, um die Denervation zu charakterisieren. In der hier abgebildeten schematischen Ansteuerung wird unterschieden zwischen der Messung von afferenten Nerven, die von der Niere hin zum zentralen Nervensystem führen, und efferenten Nerven, die vom zentralen Nervensystem hin zur Niere führen. Dabei wird ein elektrischer Stimulationspuls 481 zwischen der proximal 408_np und distal 408_nd zur Ablationselektrode 408_na angeordneten Elektroden angelegt. Parallel wird zum einen die resultierende Spannung Va von der Ablationselektrode hin zur proximal dazu angeordneten Elektrode gemessen zur Charakterisierung der Denervation der afferenten Nerven. Zum anderen wird die resultierende Spannung Ve von der Ablationselektrode hin zur distal dazu angeordneten Elektrode gemessen zur Charakterisierung der Denervation der efferenten Nerven. Dieser Mechanismus passiert unabhängig auf jeder/jedem der Rippen/Splines n, wodurch sich insgesamt die gleiche Anzahl an efferenten sowie afferenten Spannungen ergibt.

Figur 13 zeigt einen schematischen Ablauf 500 zur Durchführung einer Denervations-Prozedur. Im ersten Schritt 501 wird die Ablationskatheteranordnung intravaskulär in den Patienten eingeführt. Ist die Ablationskatheteranordnung positioniert wird durch Messung der lokalen Impedanz an den Ablations-Elektroden der Vielzahl an Splines überprüft, ob diese einen ausreichend guten Kontakt zum Gewebe aufweisen (Schritt 502). Ist dieser Kontakt für einzelne Elektroden nicht gewährleistet, so können diese selektiv durch die Steuer- und Auswerteeinheit abgeschaltet werden. Im nächsten Schritt 503 erfolgt die Aufzeichnung des Ausgangswerts der afferenten sowie efferenten Nervenleitfähigkeit durch die in Figure 12 beschriebenen Mechanismen. Daraufhin kann in Schritt 504 zwischen zwei Arten von Prozeduren gewählt werden: einer Denervation mittels gepulster Feldablation (PFA) oder einer Kombinations-Prozedur aus PFA und RF-Energie. Im Falle der PFA-Denervation (Schritt 505) erfolgt die Prozedur anhand eines wie in Figur 2 beschriebenen beispielhaften Protokolls mittels biphasischer IRE-Pulse. Wird eine PFA-RF-Kombinationsprozedur (Schritt 506) gewählt, erfolgt die Prozedur anhand eines wie in Figur 3 beispielhaft beschriebenen Kombinations-Protokolls. Unabhängig von der Wahl des Denervations-Verfahrens folgt daraufhin eine erneute Aufzeichnung der Nervenaktivität (Schritt 507) zur Charakterisierung der Denervation (Schritt 508) wie in Figur 12 beschrieben.

## Patentansprüche

1. Ablationskatheteranordnung (440; 450; 460) aufweisend:
- mindestens ein inneres Katheter-Modul (420; 430) mit einem ersten Schaft (421; 431) und einer an dem ersten Schaft (421; 431) befestigten ersten flexiblen Außenstruktur (422; 432), die ausgebildet ist, einen kollabierten Zustand und expandierten Zustand anzunehmen;
- ein äußeres Katheter-Modul (400; 410) mit einem zweiten Schaft (401; 411) und einer an dem zweiten Schaft (401; 411) befestigten zweiten flexiblen Außenstruktur, die ausgebildet ist, einen Ausgangszustand und einen Endzustand anzunehmen, wobei der zweite Schaft (401; 411) ausgebildet ist, ein inneres Katheter-Modul (420; 430) des mindestens einen inneren Katheter-Moduls (420; 430) in dem zweiten Schaft (401; 411) derart aufzunehmen, dass die zweite flexible Außenstruktur den Ausgangszustand annimmt, wenn sich die erste flexible Außenstruktur (422; 432) in dem kollabierten Zustand befindet, und dass die zweite flexible Außenstruktur den Endzustand annimmt, wenn die erste flexible Außenstruktur (422; 432) sich in dem expandierten Zustand befindet.

2. Ablationskatheteranordnung (440; 450; 460) nach Anspruch 1, wobei das mindestens eine innere Katheter-Modul (420; 430) als ein Ballon-Katheter-Modul ausgebildet ist oder ein Ballon-Katheter-Modul aufweist und die erste flexible Außenstruktur (422; 432) als eine flexible Membran ausgebildet ist oder eine flexible Membran aufweist.

3. Ablationskatheteranordnung (440; 450; 460) nach Anspruch 1 oder 2, wobei auf einer Außenseite der zweiten flexiblen Außenstruktur mehrere Elektroden (405_m) angeordnet sind.

4. Ablatationskatheteranordnung (440; 450; 460) nach einem der Ansprüche 1 bis 3, wobei die zweite flexible Außenstruktur mehrere Rippen (404_n) aufweist oder durch mehrere Rippen (404_n) gebildet ist.

5. Ablationskatheteranordnung (440; 450; 460) nach Anspruch 4, wobei an jeder der mehreren Rippen (404_n) mindestens eine Elektrode (405_m) angeordnet oder ausgebildet ist.

6. Ablationskatheteranordnung (440; 450; 460) nach einem der Ansprüche 1 bis 5, wobei die zweite flexible Außenstruktur korbförmig ausgebildet ist.

7. Ablationskatheteranordnung (440; 450; 460) nach einem der Ansprüche 1 bis 6, wobei in dem zweiten Schaft (401; 411) ein Innenschaft (406; 416) derart angeordnet und ausgebildet ist, jeweils ein inneres Katheter-Modul (420; 430) des mindestens einen inneren Katheter-Moduls (420; 430) aufzunehmen.

8. Ablationskatheteranordnung (440; 450; 460) nach einem der Ansprüche 1 bis 7, wobei das äußere Katheter-Modul (400; 410):
proximal von der zweiten flexiblen Außenstruktur eine an dem zweiten Schaft (401; 411) angebrachte oder angeordnete, beispielsweise ringförmige, Elektrode (402, 403) aufweist; und/oder
distal von der zweiten flexiblen Außenstruktur eine an dem zweiten Schaft (401; 411) angebrachte oder angeordnete, beispielsweise ringförmige, Elektrode (412, 413) aufweist.

9. Vorrichtung zur Ablation eines Gewebes eines Patienten, aufweisend:
- eine Ablationskatheteranordnung (440; 450; 460) nach einem der Ansprüche 1 bis 8;
- eine mit der Ablationskatheteranordnung (440; 450; 460) verbundene oder verbindbare Signalgeneratoranordnung; und
- eine mit der Ablationskatheteranordnung (440; 450; 460) und/oder der Signalgeneratoranordung verbundene oder verbindbare Steuer- und Auswerteeinheit.

10. Vorrichtung nach Anspruch 9, wobei die Signalgeneratoranordnung einen ersten Signalgenerator zur Erzeugung eines Radiofrequenz, RF, -Signals und einen zweiten Signalgenerator zur Erzeugung eines Signals für eine gepulste Feldablation aufweist.

11. Vorrichtung nach Anspruch 9 oder 10, wobei die Steuer- und Auswerteeinheit ausgebildet ist, den ersten Signalgenerator und/oder den zweiten Signalgenerator zur Abgabe eines Signals anzusteuern.

12. Vorrichtung nach Anspruch 11, wobei die Steuer- und Auswerteeinheit ausgebildet ist, in Abhängigkeit von mindestens einer Elektroden-Temperatur den ersten Signalgenerator und/oder den zweiten Signalgenerator zur Abgabe eines Signals anzusteuern.

13. Vorrichtung nach Anspruch 12, wobei die Steuer- und Auswerteeinheit ausgebildet ist, falls die Elektroden-Temperatur einen Temperatur-Grenzwert unterschreitet, den ersten Signalgenerator zur Abgabe eines Signals anzusteuern und, falls die Elektroden-Temperatur den Temperatur-Grenzwert annimmt oder überschreitet, den zweiten Signalgenerator zur Abgabe eines Signals anzusteuern.
